# EUROPEAN PATENT SPECIFICATION

(11) **EP 2 027 818 B1**
(45) Date of publication and mention of the grant of the patent: **09.11.2016**
(21) Application number: 07745048.4
(22) Date of filing: 11.06.2007
(51) Int. Cl.: A61B 8/12, A61B 8/00

(54) **ULTRASOUND PROBE AND ULTRASOUND ENDOSCOPE INCLUDING ULTRASOUND PROBE**
ULTRASCHALLSONDE UND ULTRASCHALLENDOSKOP MIT ULTRASCHALLSONDE
SONDE ULTRASONORE ET ENDOSCOPE ULTRASONORE DOTÉ D'UNE SONDE ULTRASONORE

(30) Priority: 12.06.2006 JP 2006162938
(43) Date of publication of application: 25.02.2009
(73) Proprietor: Olympus Corporation, Tokyo 192-8507 (JP)
(72) Inventor: SATO, Sunao, Tokyo 192-8507 (JP); WAKABAYASHI, Katsuhiro, Tokyo 192-8507 (JP); MIZUNUMA, Akiko, Tokyo 192-8507 (JP); FUJIMURA, Takanao, Tokyo 192-8507 (JP); SAWADA, Yukihiko, Tokyo 192-8507 (JP); IMAHASHI, Takuya, Tokyo 192-8507 (JP); ADACHI, Hideo, Tokyo 192-8507 (JP)
(74) Representative: von Hellfeld, Axel
(86) International application number: PCT/JP2007/061759
(87) International publication number: WO 2007/145182

(56) References cited:
- JP-A- 1 136 500
- JP-A- 4 135 400
- JP-A- 4 166 139
- JP-A- 7 274 295
- JP-A- 01 136 500
- JP-A- 01 300 937
- JP-A- 04 166 139
- JP-A- 62 048 900
- JP-A- 2001 087 265
- JP-A- 2001 087 265
- JP-A- 2005 218 518
- JP-A- 2005 510 263
- JP-U- 2 123 214
- US-A- 5 797 848
- US-A- 6 162 175

## Description

### Technical Field

The present invention relates to an ultrasound probe including a plurality of ultrasound transducers provided at a distal end of an endoscope insertion section, and an ultrasound endoscope including the ultrasound probe.

### Background Art

Ultrasound diagnostic apparatuses that transmit ultrasound to a living body such as a human body and receive echo signals reflected from living tissue to obtain tomograms of the inside of the living body can obtain image information of the inside of the living body noninvasively in real time, and are thus widely used and play an important role in diagnostic medical fields.

Ultrasound endoscopes including an ultrasound probe have been known in which a plurality of ultrasound transducers are arranged in a projecting arcuate shape, a so-called convex shape, and the ultrasound transducers are successively electronically switched at high speed for a certain time at certain intervals to obtain tomograms.

Known ultrasound endoscopes including the convex type ultrasound probe are described in, for example, Japanese Patent Application Laid-open Publication No. 8-131442 and Japanese Patent Application Laid-open Publication No. 2004-350700. The ultrasound endoscopes include an observation optical system at a distal rigid portion near the ultrasound probe, and the observation optical system has an optical axis in a front oblique direction.

The above described ultrasound probe is configured by electrically connecting a board provided with a signal pattern that transmits and receives signals to and from each ultrasound transducer among a group of a plurality of ultrasound transducers including a piezoelectric element, an acoustic matching layer, and a backside damping layer.

Such an ultrasound endoscope including the type of ultrasound probe needs to have an extremely small outer shape, and thus adjacent elements of the ultrasound transducers need to be placed at short intervals, and a plurality of signal patterns on boards connected to the ultrasound transducers placed at extremely short intervals also have to be placed at extremely short intervals.

To end electrode sections of the plurality of signal patterns on the boards, a coaxial cable having a plurality of signal lines corresponding to the plurality of ultrasound transducers are connected, and in such a device, the plurality of signal lines and pad electrodes at ends of the signal patterns need to be connected one by one, for example, by soldering.

The entire size of connecting portions between the plurality of signal lines and the pad electrodes is about several millimeters, which requires an extremely difficult connecting operation. Also, members to be connected are expensive, and loss caused by an operation error or the like significantly increases product cost.

Further, in recent years, it has been desired that an ultrasound endoscope including an ultrasound probe has a smaller diameter, and also that a printed circuit board provided with signal patterns corresponding to a plurality of ultrasound transducers has a smaller diameter.

It is supposed that the number of elements of the ultrasound transducers is reduced or intervals between the signal patterns and between the pad electrodes at the ends of the signal patterns are reduced for reducing the diameter of the printed circuit board, however, reducing the intervals is difficult as described above.

JP 1 136500 A discloses a technique for forming an ultrasonic wave probe by connecting an exciting electrode of one major face of plural piezoelectric pieces with the guide path of a 1st flexible substrate and connecting the guide path of the 1st flexible substrate to the guide path of a 2nd flexible base so as to lead the exciting electrode externally. Solder is given to a terminal region of the 1st flexible substrate from a hole of a 2nd flexible base and connected. Flexible bases are folded to the bottom face of the base, and the prolonged parts of the flexible base are overlapped and led externally. Thus, the flexible substrate is fixed to the piezoelectric plate and divided into plural piezoelectric pieces to connect the exciting electrode and the guide path. When the flexible substrate is connected to the flexible substrate, the length of the flexible substrate is prolonged.

US 6 162 175 A discloses an ultrasound transducer having a field of view greater than 180 DEG, and having a physical shape which permits it to be employed in the investigation and observation of the anatomy, or other body, object or region of interest, having limited access. A plurality of ultrasound transducer arrays are provided, each having a field of view defining an image plane, wherein the axis of each transducer array lies within its corresponding defined image plane. Preferably, the plurality of transducer arrays are positioned end-to-end and non-axially aligned with the image planes of all transducer arrays coincident, and with each transducer array having a field of view of about 90 DEG , whereby a resulting combined field of view greater than 90 DEG is produced.

US 5 797 848 A discloses an ultrasonic transducer assembly includes a shaft carried by the support element of the transducer assembly. The shaft supports an array of contacts that extend circumferentially around the shaft and are spaced axially along the length of the shaft. These contacts are electrically connected to conductors which are in turn connected to an ultrasonic transducer array. The shaft of the transducer assembly is received in a receptacle that includes a reciprocating slide that can be opened to insert and remove the transducer assembly from the receptacle. The slide can be closed to lock the transducer assembly in place, and to make electrical contact with the contacts of the shaft.

The present invention is achieved in view of the above described circumstances, and has an object to provide an ultrasound probe and an ultrasound endoscope including an ultrasound probe that allow a reduction in diameter of the ultrasound endoscope and allow reliable and easy electrical connection of signal lines extending from ultrasound transducers in the ultrasound endoscope including the ultrasound probe.

### Disclosure of Invention

### Means for Solving the Problem

A first ultrasound probe of the present invention including a plurality of ultrasound transducers provided in a distal end portion, includes: an ultrasound transducer printed circuit board provided with signal patterns that transmit and receive signals to and from the plurality of ultrasound transducers; a first electrode group that faces the plurality of ultrasound transducers and constituted by a plurality of electrode sections provided in a distal end portion of the ultrasound transducer printed circuit board; a second electrode group that is constituted by a plurality of electrode sections connected to ends of signal patterns extending from at least a part of the electrode group of the first electrode group, and arranged in a longitudinal axis direction of the ultrasound transducer printed circuit board; a first signal pattern group that is connected between each electrode section of the second electrode group and the part of the electrode sections of the first electrode group, extends from the part of the electrode sections of the first electrode group toward a proximal end side of the ultrasound transducer printed circuit board, then bends in an angle direction different from the longitudinal axis direction, and further extends toward each electrode section of the second electrode group; and a signal pattern direction changing printed circuit board provided with a second signal pattern group that has one end connectable to each electrode section of the second electrode group, extends from the one end in an angle direction different from the longitudinal axis direction, then bends in an angle direction different from the extending direction, and further extends in the longitudinal axis direction toward the proximal end portion.

A second ultrasound probe of the present invention further includes, in the first ultrasound probe, a third electrode group that is constituted by a plurality of electrode sections connected to ends of signal patterns extending from the other part of the electrode group of the first electrode group, and arranged in a width direction of the ultrasound transducer printed circuit board; and a third signal pattern group that is connected between each electrode section of the third electrode group and the other part of the electrode group of the first electrode group, and arranged in a width direction of the ultrasound transducer printed circuit board; and a third signal pattern group that is connected between each electrode section of the third electrode group and the other part of the electrode group of the first electrode group, and extends in the longitudinal axis direction toward the proximal end side of the ultrasound transducer printed circuit board.

In a third ultrasound probe of the present invention, in the first or second ultrasound probe, the plurality of ultrasound transducers are arranged in a projecting arcuate shape.

The ultrasound endoscope of the present invention includes any one of the first to third ultrasound probes at a distal end of an insertion section of an endoscope.

According to the present invention, an ultrasound probe and an ultrasound endoscope including the ultrasound probe that allows a reduction in diameter of the ultrasound probe and the ultrasound endoscope and allows reliable and easy electrical connection of signal lines extending from ultrasound transducers.

### Brief Description of the Drawings

Fig. 1 is an appearance view of an entire configuration of an ultrasound endoscope including an ultrasound probe according to a first embodiment of the present invention;
Fig. 2 is an enlarged perspective view of essential portions showing a configuration of a distal end portion of the ultrasound endoscope including the ultrasound probe according to the first embodiment;
Fig. 3 is an enlarged front view of essential portions showing the configuration of the distal end portion of the ultrasound endoscope including the ultrasound probe according to the first embodiment;
Fig. 4 is a vertical sectional view of the distal end portion of the ultrasound endoscope including the ultrasound probe according to the first embodiment;
Fig. 5 is an enlarged sectional view of essential portions of the distal end portion of the ultrasound endoscope showing an ultrasound observation region of the ultrasound endoscope including the ultrasound probe according to the first embodiment;
Fig. 6 is a plan view of an upper surface of an ultrasound transducer printed circuit board in the ultrasound endoscope including the ultrasound probe according to the first embodiment;
Fig. 7 is a plan view of a state where a predetermined relay flexible printed circuit board is connected to the ultrasound transducer printed circuit board in the ultrasound endoscope including the ultrasound probe according to the first embodiment;
Fig. 8 is a plan view of an upper surface of a relay flexible printed circuit board for changing a signal pattern direction connected to the ultrasound transducer printed circuit board in the ultrasound endoscope including the ultrasound probe according to the first embodiment;
Fig. 9 is a plan view of a state where a coaxial cable group is connected to the relay flexible printed circuit board for changing a signal pattern direction connected to the ultrasound transducer printed circuit board in the ultrasound endoscope including the ultrasound probe according to the first embodiment;
Fig. 10 is a plan view of a state where a plurality of predetermined relay flexible printed circuit boards are connected to the ultrasound transducer printed circuit board in the ultrasound endoscope including the ultrasound probe according to the first embodiment;
Fig. 11 is a plan view of an upper surface of a printed circuit board provided with signal lines corresponding to a plurality of ultrasound transducers in an ultrasound endoscope including an ultrasound probe according to a second embodiment of the present invention;
Fig. 12 is a plan view of an upper surface of a first relay flexible printed circuit board for changing a signal pattern direction connected to an ultrasound transducer printed circuit board in the ultrasound endoscope including the ultrasound probe according to the second embodiment;
Fig. 13 is a plan view of a state where the first relay flexible printed circuit board for changing a signal pattern direction is connected to the ultrasound transducer printed circuit board in the ultrasound endoscope including the ultrasound probe according to the second embodiment;
Fig. 14 is a plan view of a state where a plurality of predetermined relay flexible printed circuit boards for changing a signal pattern direction are connected to the ultrasound transducer printed circuit board in the ultrasound endoscope including the ultrasound probe according to the second embodiment;
Fig. 15 is a plan view of an upper surface of a printed circuit board provided with signal lines corresponding to a plurality of ultrasound transducers in an ultrasound endoscope including an ultrasound probe according to a third embodiment of the present invention;
Fig. 16 is a sectional view taken along the line XVI-XVI in Fig. 10;
Fig. 17 is an appearance perspective view of an engagement state between the ultrasound transducer printed circuit board and an ultrasound probe unit in the first embodiment;
Fig. 18 is an appearance perspective view of an engagement state between the ultrasound transducer printed circuit board and the ultrasound probe unit in a variant of the first embodiment; and
Fig. 19 is an enlarged sectional view of essential portions of the engagement state between the ultrasound transducer printed circuit board and the ultrasound probe unit in the variant of the first embodiment.

### Best Mode for Carrying Out the Invention

Now, the present embodiments will be described with reference to the drawings.

The present invention provides an ultrasound endoscope including an ultrasound probe having features in a printed circuit board provided with signal lines corresponding to a plurality of ultrasound transducers as patterns and a flexible printed circuit board connected to the printed circuit board in the ultrasound probe. Prior to the description on the boards, the ultrasound endoscope including the ultrasound probe having the boards and the like will be described.

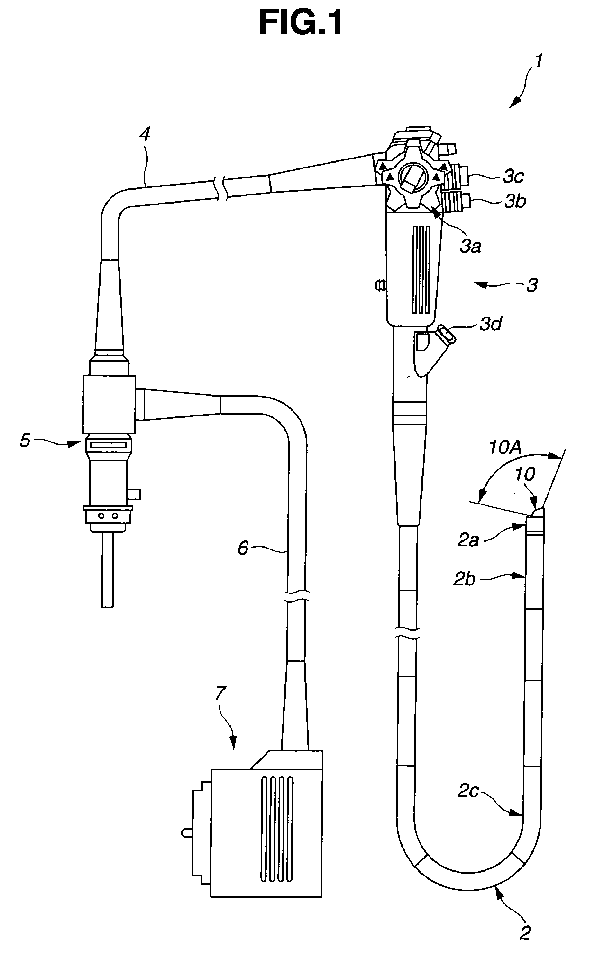

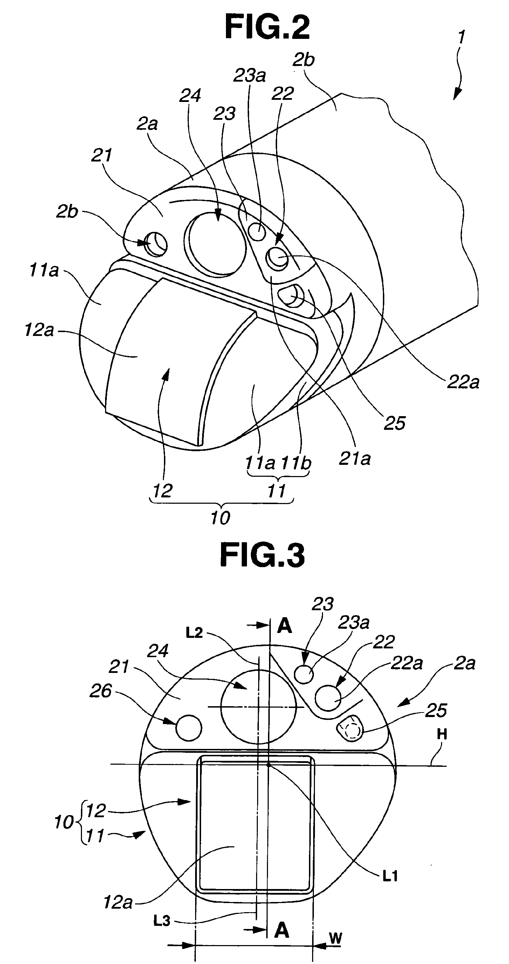

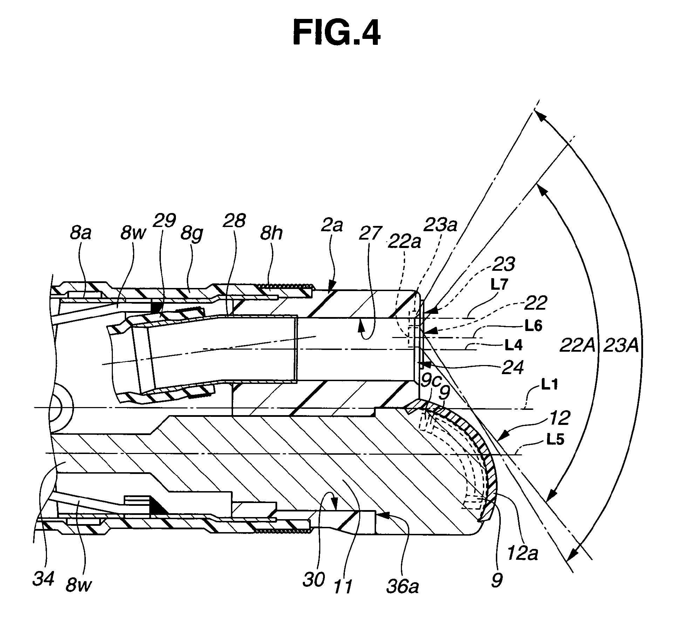

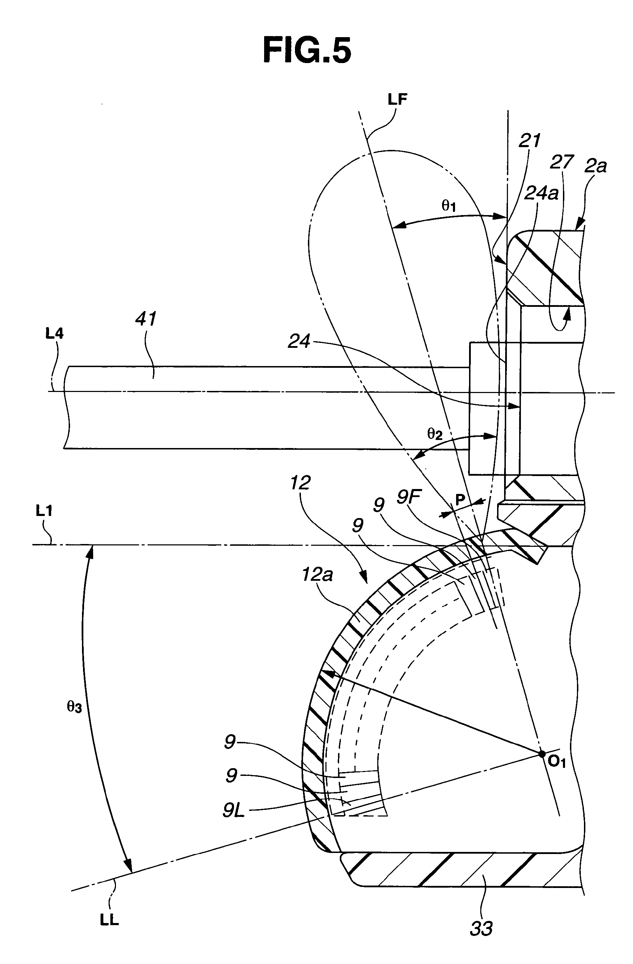

Fig. 1 is an appearance view of an entire configuration of an ultrasound endoscope including an ultrasound probe according to a first embodiment of the present invention, Fig. 2 is an enlarged perspective view of essential portions showing a configuration of a distal end portion of the ultrasound endoscope including the ultrasound probe according to the first embodiment, Fig. 3 is an enlarged front view of essential portions showing the configuration of the distal end portion of the ultrasound endoscope including the ultrasound probe according to the first embodiment, Fig. 4 is a vertical sectional view of the distal end portion of the ultrasound endoscope including the ultrasound probe according to the first embodiment, and Fig. 5 is an enlarged sectional view of essential portions of the distal end portion of the ultrasound endoscope showing an ultrasound observation region of the ultrasound endoscope including the ultrasound probe according to the first embodiment.

As shown in Fig. 1, an ultrasound endoscope (hereinafter also referred to as an endoscope) 1 of the present embodiment includes an elongated insertion section 2 inserted into a body cavity, an operation section 3 provided at a proximal end of the insertion section 2, and a universal cord 4 extending from a side portion of the operation section 3.

An endoscope connector 5 is provided at the other end of the universal cord 4. An ultrasound cable 6 extends from a side portion of the endoscope connector 5. An ultrasound connector 7 is provided at the other end of the ultrasound cable 6.

The insertion section 2 includes a distal rigid portion 2a formed of a rigid member, a bending portion 2b configured to be bendable, and a flexible tube portion 2c that is long and flexible and extends from a proximal end of the bending portion 2b to a distal end of the operation section 3 in order from a distal end side.

On the operation section 3, an angle knob 3a for a bending operation is provided. Also on the operation section 3, an air/water feeding button 3b for feeding air and water, and a suction button 3c for suction are provided. Further, in the operation section 3, a treatment instrument insertion opening 3d for introducing a treatment instrument into a body cavity is provided.

Reference numeral 10 denotes an ultrasound unit including an ultrasound probe having a convex type ultrasound scanning surface. The ultrasound unit 10 forms an ultrasound scanning range 10A for scanning a forward direction in an endoscope insertion axis direction.

As shown in Figs. 2 and 3, the ultrasound unit 10 for obtaining acoustic image information by ultrasound is provided in the distal rigid portion 2a of the insertion section 2. The ultrasound unit 10 includes a nose piece 11 that is a casing and an ultrasound probe 12. The ultrasound probe 12 is provided integrally at a notch portion formed substantially in the middle of the nose piece 11. As shown in the drawings, a tissue contact surface 11a that constitutes the nose piece 11 and an acoustic lens surface 12a of the ultrasound probe 12 project from a distal end surface 21 of the distal rigid portion 2a.

As shown in Figs. 4 and 5, the ultrasound probe 12 includes a plurality of ultrasound transducers 9 arranged in a projecting arcuate shape, a so-called convex shape, the acoustic lens 12a, and an unshown electric wire, and when the ultrasound probe 12 is connected to an observation device, an ultrasound image is obtained.

On the other hand, as shown in Figs. 2 and 3, in the distal end surface 21 of the distal rigid portion 2a, an observation window 22a that constitutes an observation optical system 22, an illumination window 23a that constitutes an illumination optical system 23, a treatment instrument lead-out opening 24 through which a treatment instrument such as a puncture needle is led out, an air/water feeding nozzle 25 that ejects a fluid such as water or air toward the observation window 22a, and a sub-water feeding channel opening 26 for feeding water forward. Instead of providing the sub-water feeding channel opening 26, the sub-water feeding channel opening 26 may be configured as a second treatment instrument lead-out opening.

A central axis of the treatment instrument lead-out opening 24 is arranged in line with a centerline L2 of the ultrasound probe 12 so that the treatment instrument led out of the treatment instrument lead-out opening 24 is placed within the ultrasound scanning range 10A obtained by the ultrasound probe 12.

The observation window 22a, the illumination window 23a, and the air/water feeding nozzle 25 are collectively placed, for example, on the right side in the drawings, relative to the treatment instrument lead-out opening 24, and outside the ultrasound scanning range 10A. Among the observation window 22a, the illumination window 23a, and the air/water feeding nozzle 25, the air/water feeding nozzle 25 is placed at the most distant position from the ultrasound observation region 10A. In the present embodiment, the illumination window 23a, the observation window 22a, and the air/water feeding nozzle 25 are placed in line in view of improvement in observation performance, improvement in cleaning property, and reduction in outer diameter of the distal end portion of the endoscope.

The observation window 22a has an observation field range (see a range of reference numeral 22A shown by dash-single-dot lines in Fig. 4) of the observation optical system 22. The illumination window 23a has an illumination light application range (see a range of reference numeral 23A shown by dash-double-dot lines in Fig. 4) of the illumination optical system 23. The observation field range 22A and the illumination light application range 23A are formed so as not to include the ultrasound probe 12.

The observation window 22a and the illumination window 23a are provided in an observation distal end surface 21a slightly projecting from the distal end surface 21. The sub-water feeding channel opening 26 is placed on the other side opposite from one side provided with the observation window 22a, the illumination window 23a, and the air/water feeding nozzle 25 with the treatment instrument lead-out opening 24 therebetween, and outside the ultrasound scanning range 10A. When the sub-water feeding channel opening 26 is formed as the second treatment instrument lead-out opening, a diameter of the channel is set according to a treatment instrument to be used.

This allows a procedure using two treatment instruments to be performed under observation by the endoscope. Thus, the treatment instrument projected through the second treatment instrument lead-out opening and used under observation by the endoscope, and the treatment instrument projected through the treatment instrument lead-out opening 24 and used under ultrasound diagnosis are combined to allow efficient diagnosis and medical treatment.

As shown in Fig. 4, a distal bending piece 8a that constitutes the bending portion 2b is connected and secured to a proximal end side of the distal rigid portion 2a. A plurality of bending pieces (not shown) are connected to the distal bending piece 8a. A centerline of the bending portion 2b constituted by the connected bending pieces is an endoscope insertion axis L1.

To predetermined positions on the distal bending piece 8a, distal end portions of top, bottom, left and right bending wires 8w are secured. Thus, an operator operates the angle knob 3a to pull or loosen a bending wire 8w corresponding to the operation, and thus the bending portion 2b performs a bending operation. The plurality of bending pieces are coated with bending rubber 8g. A distal end portion of the bending rubber 8g is integrally secured by a bobbin bonding section 8h provided in the distal rigid portion 2a.

The distal end surface 21 and the observation distal end surface 21a of the distal rigid portion 2a are formed perpendicularly to the endoscope insertion axis L1. In the distal rigid portion 2a, a treatment instrument passing channel hole (hereinafter referred to as a treatment instrument hole) 27 that constitutes the treatment instrument lead-out opening 24, and a positioning hole 30 are formed.

In the distal rigid portion 2a, besides the holes 27 and 30, a through hole in which the observation optical system is provided, a through hole in which the illumination optical system is provided, a through hole for air/water feeding for supplying the fluid ejected from the air/water feeding nozzle 25, a through hole that constitutes the sub-water feeding channel opening 26, or the like are provided, though not shown.

A longitudinal central axis L4 of the treatment instrument hole 27 is formed substantially in parallel with the endoscope insertion axis L1. A longitudinal central axis L5 of the positioning hole 30 is formed substantially in parallel with the endoscope insertion axis L1. An optical axis L6 of the observation optical system and an optical axis L7 of the illumination optical system provided in the ultrasound endoscope 1 are also formed in parallel with the endoscope insertion axis L1.

Thus, the observation optical system provided in the ultrasound endoscope 1 of the present embodiment is of a so-called forward viewing type in which an observation field is set in a forward front, in other words, in an insertion direction forward of the endoscope insertion axis L1.

With a proximal end side of the treatment instrument hole 27, one end of a tube connection pipe 28 formed to be tilted by a predetermined amount communicates. The tube connection pipe 28 communicates at the other end with one end of a channel tube 29 that constitutes a treatment instrument passing channel. The other end of the channel tube 29 communicates with the treatment instrument insertion opening 3d.

The treatment instrument inserted through the treatment instrument insertion opening 3d smoothly moves in the channel tube 29, the tube connection pipe 28, and the treatment instrument hole 27, and is led out of the treatment instrument lead-out opening 24. The treatment instrument led out of the treatment instrument lead-out opening 24 is led out forward that is the insertion direction of the insertion section 2, in parallel with the endoscope insertion axis L1.

Specifically, with, for example, a distal end portion of a puncture needle as a treatment instrument placed in the treatment instrument hole 27, a needle tube that constitutes the puncture needle is projected. Then, the needle tube is projected from the treatment instrument lead-out opening 24 to the forward front observed through the observation window 22a, substantially in parallel with the endoscope insertion axis L1.

Meanwhile, the positioning hole 30 is provided in the distal rigid portion 2a. The ultrasound unit 10 fits in the positioning hole 30, and a contact surface of the nose piece 11 and an abutment surface 36 of the distal rigid portion 2a contact each other to position the ultrasound unit 10 in the positioning hole 30. From the other end of the ultrasound unit 10, an ultrasound cable 34 connected to the ultrasound probe 12 is led out.

An outer shape from the abutment surface 36 to the distal end of the distal rigid portion 2a (a surface of reference numeral 11b in Fig. 2) has an elevation width W of the ultrasound probe 12 and the contact surface 11a, and has substantially the same size as an outer size of the distal end of the distal rigid portion 2a as shown in Fig. 2.

Thus, when the ultrasound unit 10 is abutted against body tissue in ultrasound observation, a force of the operator gripping the operation section 3 in the direction of the endoscope insertion axis L1 is reliably transmitted to the ultrasound unit 10. This allows the tissue contact surface 11 a and the acoustic lens surface 12a to be substantially uniformly brought into close contact with the body tissue. Thus, the tissue contact surface 11a and the acoustic lens surface 12a of the ultrasound unit 10 can be stably abutted against the body tissue to obtain a good ultrasound observation image.

The ultrasound probe 12 in Figs. 4 and 5 is configured by arranging a plurality of ultrasound transducers 9 including, for example, a packing material, a piezoelectric transducer, a matching layer, and an acoustic lens stacked, and providing an electric wire such as a cable.

The plurality of ultrasound transducers 9 are arranged from a first ultrasound transducer 9F that is placed closest to the treatment instrument projecting opening and emits ultrasound to a final ultrasound transducer 9L placed the most distant from the treatment instrument projecting opening at a predetermined interval p. As shown in Fig. 5, the center of curvature O1 of the arc of the ultrasound probe 12 is positioned closer to the proximal end side than an opening surface 24a of the treatment instrument lead-out opening 24 provided in the distal rigid portion 2a. In the ultrasound transducer 9, an MUT (Micromachined Ultrasound Transducer) element may be used instead of the piezoelectric element.

Thus, the center of curvature O1 of the arc of the ultrasound probe 12 is positioned closer to the proximal end side than the opening surface 24a of the treatment instrument lead-out opening 24 to reduce the length of the distal rigid portion of the endoscope 1. This improves an insertion property of the endoscope into the body cavity. The ultrasound probe 12 is not placed within the observation field range of the endoscope 1, thereby eliminating inconvenience that a part of an endoscope image is lost by the ultrasound probe 12. Further, the ultrasound probe 12 is not placed within the illumination light application range of the endoscope 1, and thus a part of illumination light is not blocked by the ultrasound probe 12, and the illumination light can be applied over the observation field range of the endoscope 1 to obtain a good endoscope image.

In the ultrasound probe 12, a direction of a central axis LF of a sound ray of the first ultrasound transducer 9F is tilted by an angle θ1 toward the distal end side relative to the distal end surface 21 with reference to the distal end surface 21 (specifically, the distal end surface 21 having the treatment instrument lead-out opening 24) of the distal rigid portion 2a.

Next, a printed circuit board provided with signal lines corresponding to the plurality of ultrasound transducers as patterns, and a flexible printed circuit board connected to the printed circuit board in the ultrasound endoscope including the ultrasound probe according to the first embodiment of the present invention will be described.

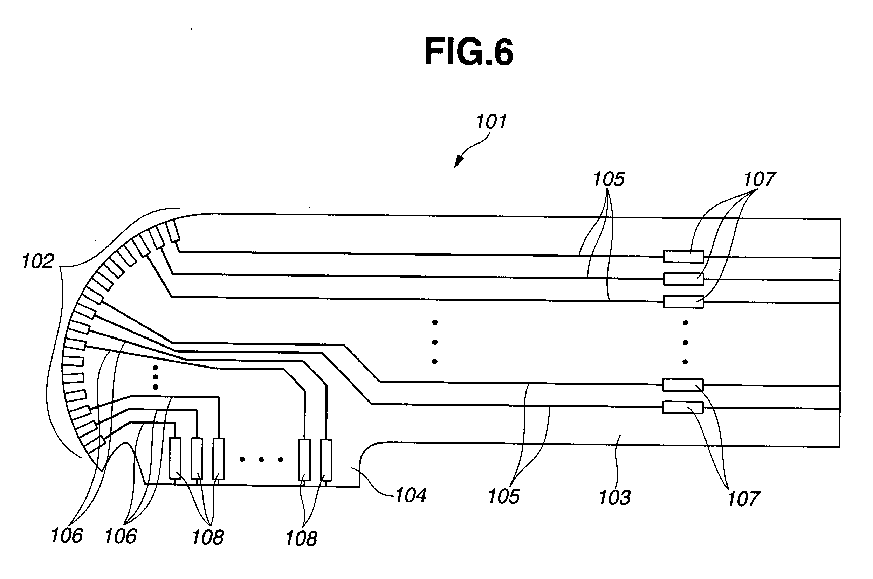

Fig. 6 is a plan view of an upper surface of a printed circuit board provided with signal lines corresponding to the plurality of ultrasound transducers (hereinafter referred to as an ultrasound transducer printed circuit board) in the ultrasound endoscope including the ultrasound probe according to the first embodiment of the present invention.

As shown, an ultrasound transducer printed circuit board 101 is a long and substantially rectangular printed circuit board provided in an insertion section axial direction in the distal rigid portion 2a of the insertion section 2 of the ultrasound endoscope, and is preferably a printed circuit board formed of a rigid member for use as a positioning member with high accuracy in assembly of the ultrasound probe.

On a distal end side of the ultrasound transducer printed circuit board 101, a group of a plurality of transducer wiring pads 102 facing the plurality of ultrasound transducers 9 arranged in the convex shape are arranged in a convex shape according to the arrangement of the ultrasound transducers 9.

From the group of the plurality of transducer wiring pads 102, a first signal pattern group 105 and a second signal pattern group 106 extend toward the proximal end side of the ultrasound transducer printed circuit board 101.

The first signal pattern group 105 is connected at one end to pads of about a half of the group on one side of the board of the transducer wiring pad group 102, and then extends in a longitudinal axis direction of the ultrasound transducer printed circuit board 101 toward a proximal end portion 103 of the ultrasound transducer printed circuit board 101.

In the proximal end portion 103, a first flexible printed circuit board wiring pad group 107 connected to the other end of the first signal pattern group 105 is arranged in a width direction of the ultrasound transducer printed circuit board 101 (in a short axis direction, that is, a direction substantially perpendicular to the longitudinal axis direction of the ultrasound transducer printed circuit board 101; a direction perpendicular to an insertion axis of the endoscope). The first flexible printed circuit board wiring pad group 107 is arranged within a range of several millimeters with reduction in diameter of the ultrasound endoscope, and provided at a distance reliably connectable to a plurality of signal lines on a flexible printed circuit board described later.

Closer to the proximal end side than the first flexible printed circuit board wiring pad group 107, a signal pattern for checking in production is provided.

Meanwhile, the second signal pattern group 106 is connected at one end to pads of about a half of the group on the other side of the board of the transducer wiring pad group 102, then once extends in the longitudinal axis direction of the ultrasound transducer printed circuit board 101, and then bends substantially at 90 degrees and extends toward a distal projecting portion 104 in the ultrasound transducer printed circuit board 101.

The distal projecting portion 104 is formed along a side surface of the board that has no influence on the arrangement of the first flexible printed circuit board wiring pad group 107 in the ultrasound transducer printed circuit board 101. In the distal projecting portion 104, a second flexible printed circuit board wiring pad group 108 connected to the other end of the second signal pattern group 106 is arranged in the longitudinal axis direction of the ultrasound transducer printed circuit board 101.

The second flexible printed circuit board wiring pad group 108 is provided at a distance reliably connectable to the plurality of signal lines on the flexible printed circuit board described later like the first flexible printed circuit board wiring pad 107, but the second flexible printed circuit board wiring pad group 108 is arranged in the longitudinal axis direction of the ultrasound transducer printed circuit board 101 and thus can be placed relatively with space unlike the first flexible printed circuit board wiring pad 107.

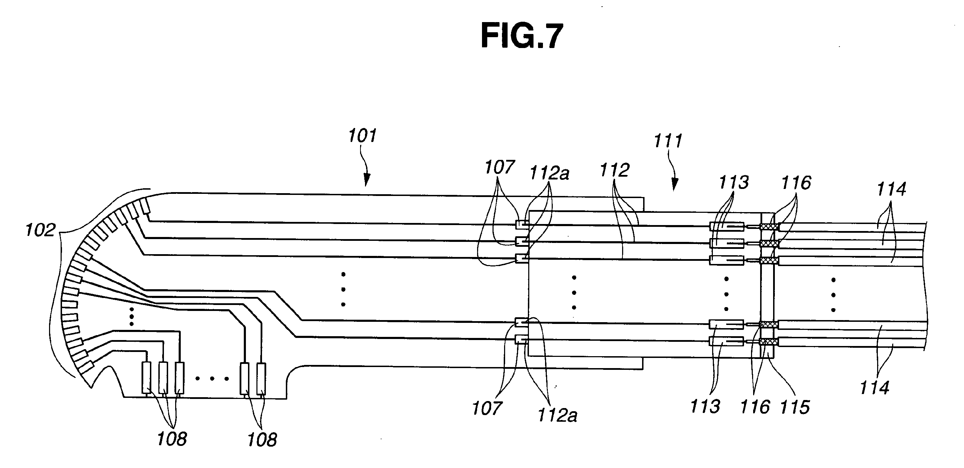

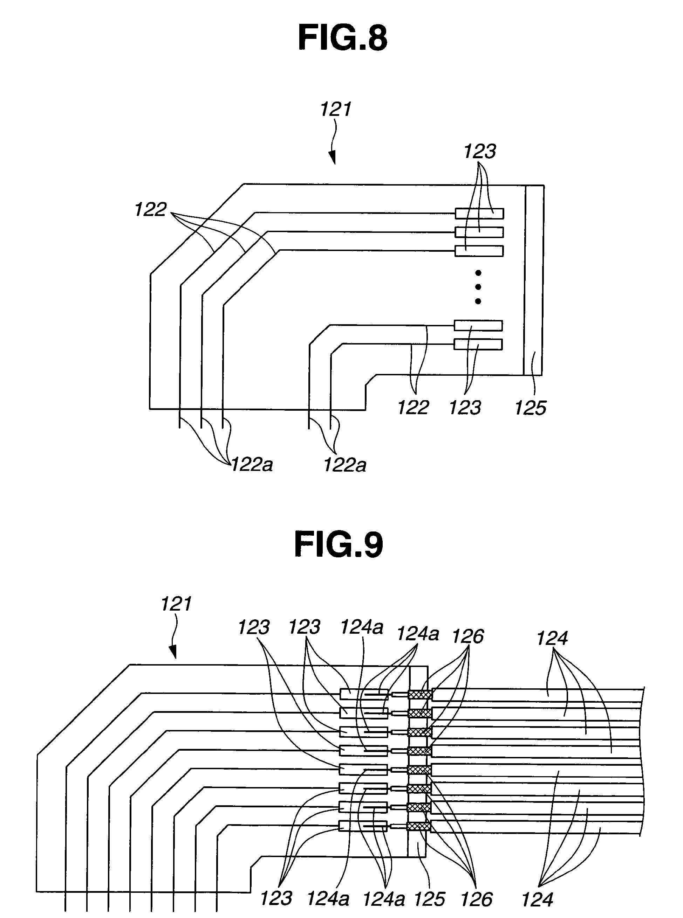

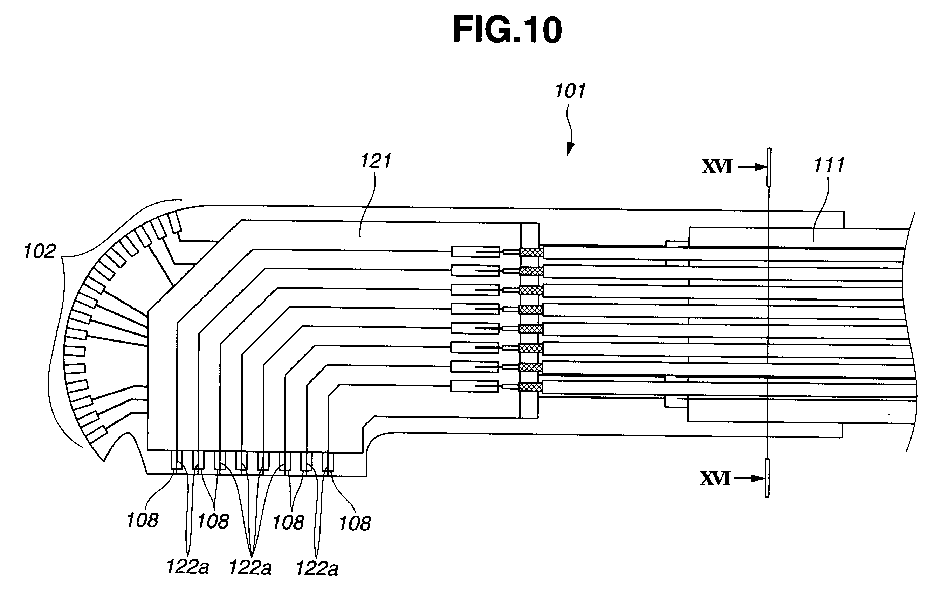

Fig. 7 is a plan view of a state where a predetermined relay flexible printed circuit board is connected to the ultrasound transducer printed circuit board in the ultrasound endoscope including the ultrasound probe according to the first embodiment of the present invention, Fig. 8 is a plan view of an upper surface of a relay flexible printed circuit board for changing a signal pattern direction connected to the ultrasound transducer printed circuit board in the ultrasound endoscope including the ultrasound probe according to the first embodiment of the present invention, Fig. 9 is a plan view of a state where a coaxial cable group is connected to the relay flexible printed circuit board for changing a signal pattern direction connected to the ultrasound transducer printed circuit board in the ultrasound endoscope including the ultrasound probe according to the first embodiment of the present invention, and Fig. 10 is a plan view of a state where a plurality of predetermined relay flexible printed circuit boards are connected to the ultrasound transducer printed circuit board in the ultrasound endoscope including the ultrasound probe according to the first embodiment of the present invention.

In the ultrasound endoscope, an unshown signal cable electrically connected to the ultrasound connector 7 (see Fig. 1) passes through the insertion section 2 and reaches near the ultrasound transducer printed circuit board 101. The signal cable 34 (see Fig. 4) is preferably constituted by a plurality of coaxial cables corresponding to the plurality of ultrasound transducers 9.

As shown in Fig. 7, the ultrasound endoscope of the present embodiment includes a first coaxial cable group 114 that includes a part of signal cables of the plurality of coaxial cables and corresponds to the first signal pattern group 105, and a first relay flexible printed circuit board 111 that relays the first coaxial cable group 114 and the first signal pattern group 105.

The first relay flexible printed circuit board 111 has a rectangular shape with a smaller width than the ultrasound transducer printed circuit board 101, and is provided with a signal pattern group 112 of the same number as the first signal pattern group 105. A distal end of the signal pattern group 112 is a flying lead section 112a projecting from an end surface of the board by a so-called flying lead structure, and is connectable to the first flexible printed circuit board wiring pad group 107 by soldering or the like. On the other hand, a first coaxial cable wiring pad group 113 for connecting the first coaxial cable group 114 is arranged on the other end of the signal pattern group 112.

Shield wires (ground wires) 116 of the first coaxial cable group 114 are collectively connected to a ground pad 115 of the first relay flexible printed circuit board 111 by soldering or the like.

In the present embodiment, the first relay flexible printed circuit board 111 on which a core line (signal line) of the first coaxial cable group 114 is previously connected to the first coaxial cable wiring pad 113 is connected to the ultrasound transducer printed circuit board 101. Specifically, the flying lead section 112a is connected to the first flexible printed circuit board wiring pad group 107 by soldering or the like.

As shown in Figs. 8 and 9, the ultrasound endoscope of the present embodiment includes a second coaxial cable group 124 that is a part of signal cables of the plurality of coaxial cables and corresponds to the second signal pattern group 106, and a second relay flexible printed circuit board 121 that relays the second coaxial cable group 124 and the second signal pattern group 106.

The second relay flexible printed circuit board 121 has a proximal end portion having a rectangular shape with the same width as or a slightly smaller width than the ultrasound transducer printed circuit board 101, and a distal end portion slightly projecting toward a side surface of the proximal end portion, and thus forms a substantially L shape.

The second relay flexible printed circuit board 121 is provided with a signal pattern group 122 of the same number as the second signal pattern group 106 along the substantially L shape. A distal end of the signal pattern group 122 is formed with a flying lead section 122a similarly to the above, and is connectable to the second flexible printed circuit board wiring pad group 108 by soldering or the like.

The signal pattern group 122 once extends from a connecting portion to the second flexible printed circuit board wiring pad group 108, that is, the flying lead section 122a toward one side of the board, then bends at substantially 90 degrees in the middle of the signal pattern, and extends toward the proximal end portion of the board. Then, a second coaxial cable wiring pad group 123 for connecting a core line of the second coaxial cable group 124 is arranged in the width direction (short axis direction) of the board on the other end of the signal pattern group 122.

Shield wires (ground wires) 126 of the second coaxial cable group 124 are collectively connected to a ground pad 125 of the second relay flexible printed circuit board 121 by soldering or the like.

Thus, the signal pattern group 122 changes a direction of the signal pattern extending from the second flexible printed circuit board wiring pad group 108 arranged in the longitudinal axis direction of the ultrasound transducer printed circuit board 101 (a direction at an angle of 90 degrees relative to the longitudinal axis direction) into the longitudinal axis direction of the ultrasound transducer printed circuit board 101.

Specifically, the second relay flexible printed circuit board 121 performs a direction changing function of changing the direction of the signal pattern from the second flexible printed circuit board wiring pad group 108 that can be relatively placed with space into the longitudinal direction of the ultrasound transducer printed circuit board 101 (axial direction of the insertion section 2).

In the present embodiment, the first relay flexible printed circuit board 111 is connected to the ultrasound transducer printed circuit board 101 (the state in Fig. 7), and then the second relay flexible printed circuit board 121 on which the second coaxial cable group 124 is previously connected to the second coaxial cable wiring pad group 123 is connected to the ultrasound transducer printed circuit board 101 (the state in Fig. 10). Specifically, the flying lead section 122a is connected to the second flexible printed circuit board wiring pad group 108 by soldering or the like.

In the above described example, the ultrasound transducer printed circuit board 101 is provided with the signal pattern and the wiring pad described above only on the upper surface as shown in Fig. 6, but not being limited thereto, components similar to the signal pattern and the wiring pad may be provided symmetrically on a back surface. Specifically, the board may be configured as a double-sided board, not limited to the single-sided board.

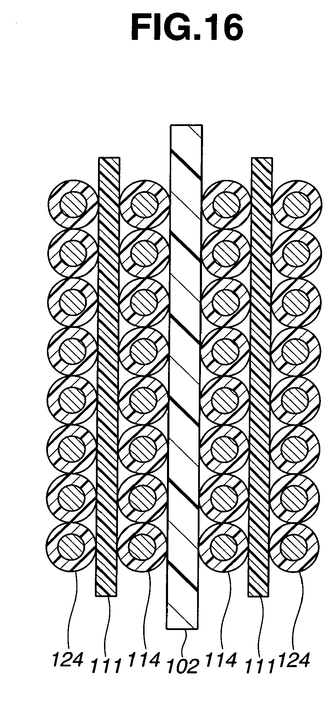

When the board is configured as a double-sided board, the first relay flexible printed circuit board 111 and the second relay flexible printed circuit board 121 may be connected to both sides, and at this time, a section taken along the line XVI-XVI in Fig. 10 has a configuration as shown in Fig. 16.

As described above, in the ultrasound endoscope using the ultrasound probe of the first embodiment,
(1) the plurality of signal patterns extending from the plurality of ultrasound transducers are divided into the two groups on the ultrasound transducer printed circuit board,
(2) one signal pattern group extends in the longitudinal axis direction of the ultrasound transducer printed circuit board, then the pad electrode section in the distal end portion is arranged in the short axis direction of the board, and the signal line extending from the pad electrode section toward the proximal end side extends in the longitudinal axis direction, and
(3) the other signal pattern group once extends in the longitudinal axis direction of the ultrasound transducer printed circuit board and then bends at substantially 90 degrees, the pad electrode section in the distal end portion is arranged in the longitudinal axis direction of the board, and the direction of the signal line extending from the pad electrode section in the short axis direction is changed into the longitudinal axis direction by the direction changing board.

This can provide the ultrasound probe and the ultrasound endoscope including the ultrasound probe that allows a reduction in diameter of the ultrasound probe and the ultrasound endoscope and allows reliable and easy electrical connection of the signal lines extending from the ultrasound transducers.

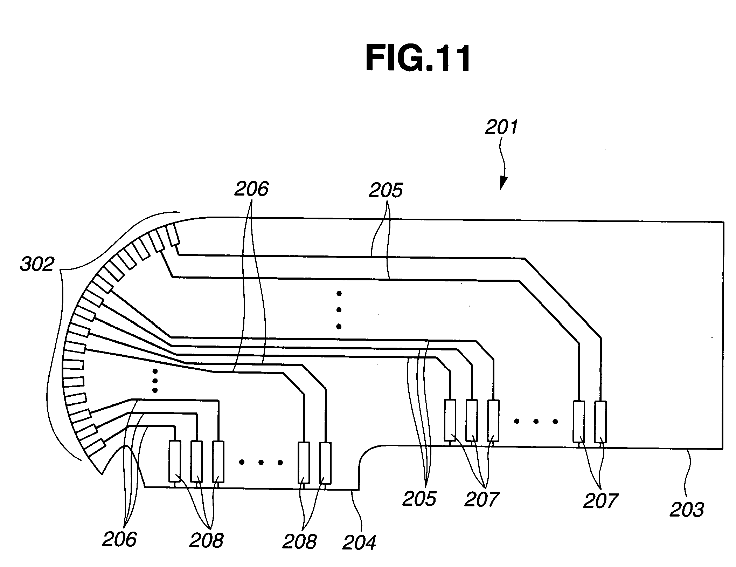

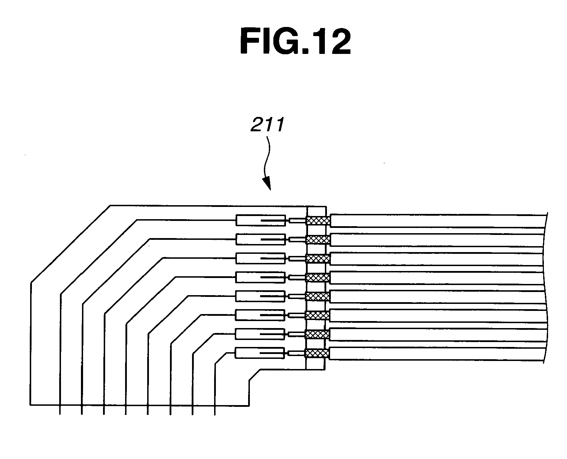

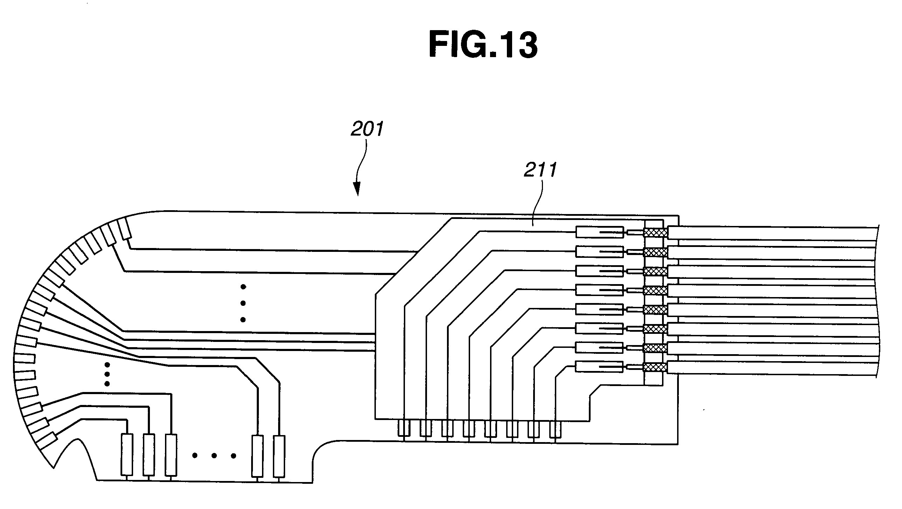

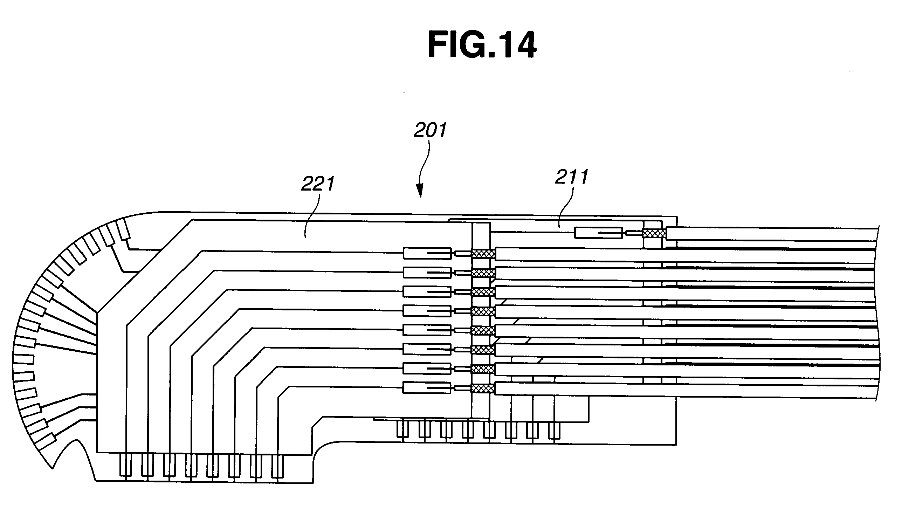

Next, a second embodiment of the present invention will be described. Fig. 11 is a plan view of an upper surface of a printed circuit board provided with signal lines corresponding to a plurality of ultrasound transducers in an ultrasound endoscope including an ultrasound probe according to a second embodiment of the present invention, Fig. 12 is a plan view of an upper surface of a first relay flexible printed circuit board for changing a signal pattern direction connected to the ultrasound transducer printed circuit board in the ultrasound endoscope including the ultrasound probe according to the second embodiment of the present invention, Fig. 13 is a plan view of a state where the first relay flexible printed circuit board for changing a signal pattern direction is connected to the ultrasound transducer printed circuit board in the ultrasound endoscope including the ultrasound probe according to the second embodiment of the present invention, and Fig. 14 is a plan view of a state where a plurality of predetermined relay flexible printed circuit boards for changing a signal pattern direction are connected to the ultrasound transducer printed circuit board in the ultrasound endoscope including the ultrasound probe according to the second embodiment of the present invention.

As shown in Fig. 11, an ultrasound transducer printed circuit board 201 in the second embodiment has the same function as the ultrasound transducer printed circuit board 101 in the first embodiment, but unlike the first embodiment, the ultrasound transducer printed circuit board 201 has a feature in that both of two divided signal pattern groups once extend in the longitudinal axis direction and then bend at an angle of substantially 90 degrees, pad electrode sections at distal end portions are arranged in the longitudinal axis direction, and directions of signal lines extending from the pad electrode sections in the short axis direction are changed into the longitudinal axis direction by a direction changing board in both of the signal pattern groups.

Other configurations are the same as in the first embodiment, and thus differences only will be herein described.

On a distal end side of the ultrasound transducer printed circuit board 201 in the second embodiment, a group of a plurality of transducer wiring pads 202 similar to the transducer wiring pad group 102 are arranged, and from the group of the plurality of transducer wiring pads 202, a first signal pattern group 205 and a second signal pattern group 206 extend toward a proximal end side of the ultrasound transducer printed circuit board 201.

The first signal pattern group 205 is connected at one end to pads of about a half of the group on one side of the board of the transducer wiring pad group 202, then extends in a longitudinal axis direction of the ultrasound transducer printed circuit board 201 toward a proximal end portion 203, and bends at substantially 90 degrees toward the other side in the proximal end portion 203.

In the proximal end portion 203, a first flexible printed circuit board wiring pad group 207 connected to the other end of the first signal pattern group 205 is arranged in the longitudinal axis direction of the ultrasound transducer printed circuit board 201.

Meanwhile, the second signal pattern group 206 is connected at one end to pads of about a half of the group on the other side of the board of the transducer wiring pad group 202, then once extends in the longitudinal axis direction of the ultrasound transducer printed circuit board 201, and then bends substantially at 90 degrees and extends toward a distal projecting portion 204 in the ultrasound transducer printed circuit board 201.

In the distal projecting portion 204, a second flexible printed circuit board wiring pad group 208 similar to the second flexible printed circuit board wiring pad group 108 in the first embodiment is arranged in the longitudinal axis direction of the ultrasound transducer printed circuit board 201.

As shown in Figs. 12 and 13, a first relay flexible printed circuit board 211 (see Fig. 12) that plays the same role as the second relay flexible printed circuit board 121 in the first embodiment is connected to the first flexible printed circuit board wiring pad group 207 (see Fig. 13).

Further, as shown in Fig. 14, a second relay flexible printed circuit board 221 that plays the same role as the second relay flexible printed circuit board 121 in the first embodiment is connected to the second flexible printed circuit board wiring pad group 208. At this time, as in the first embodiment, the first relay flexible printed circuit board 211 is connected and then the second relay flexible printed circuit board 221 is connected.

As described above, in the ultrasound endoscope of the second embodiment,
(1) the plurality of signal patterns extending from the plurality of ultrasound transducers are divided into the two groups on the ultrasound transducer printed circuit board,
(2) both of the signal pattern groups once extend in the longitudinal axis direction of the ultrasound transducer printed circuit board and then bend at substantially 90 degrees, the pad electrode sections in the distal end portions are arranged in the longitudinal axis direction of the board, and the directions of the signal lines extending from the pad electrode sections in the short axis direction are changed into the longitudinal axis direction by the direction changing board.

This allows a further reduction in diameter of the ultrasound endoscope as compared with the first embodiment depending on the shape of the ultrasound transducer printed circuit board.

Also in the second embodiment, the board may be configured as a double-sided board as in the first embodiment.

Further, in the above described ultrasound endoscope of the first and second embodiments, the signal pattern group bends at the angle of substantially 90 degrees, but the angle is not limited thereto as long as the signal pattern group can be effectively connected to the flexible printed circuit board wiring pad group provided in the longitudinal axis direction of the ultrasound transducer printed circuit board, and the above described advantage can be obtained, for example, at an angle of about 60 degrees.

Further, in the ultrasound endoscopes of the first and second embodiments, the signal patterns extending from the ultrasound transducers are divided into the two groups, but not being limited thereto, the signal patterns may be divided into three or more groups. In this case, it should be understood that a signal pattern group with an angle changed and a signal pattern group with an angle unchanged may be combined in any manner in view of the size and shape of the ultrasound transducer printed circuit board and cost, or the like.

Next, a third embodiment of the present invention will be described.

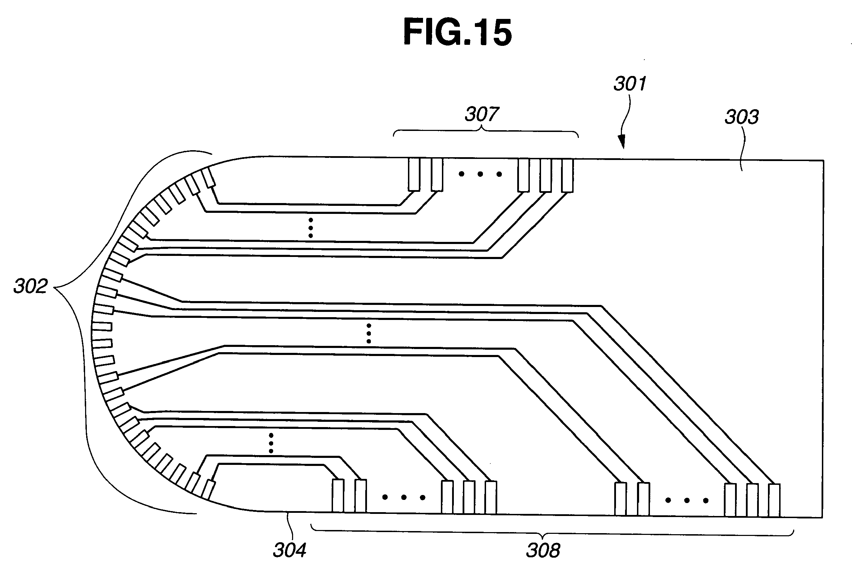

Fig. 15 is a plan view of an upper surface of a printed circuit board provided with signal lines corresponding to a plurality of ultrasound transducers in an ultrasound endoscope including an ultrasound probe according to a third embodiment of the present invention.

As shown in Fig. 15, an ultrasound transducer printed circuit board 301 in the third embodiment has the same function as the ultrasound transducer printed circuit board 101 in the first embodiment in terms of providing signal patterns from ultrasound transducers, but unlike the first embodiment, the ultrasound transducer printed circuit board 301 has a feature in that a plurality of signal pattern groups separately bend toward both sides of the board, and pad electrode sections in distal end portions are also arranged in a longitudinal axis direction separately on both sides of the board, and directions of signal lines extending from the pad electrode sections in the short axis direction are changed into the longitudinal axis direction by a direction changing board in the plurality of signal pattern groups.

Other configurations are the same as in the first embodiment, and thus differences only will be herein described.

On a distal end side of the ultrasound transducer printed circuit board 301 in the third embodiment, a group of a plurality of transducer wiring pads 302, which has a different shape from the transducer wiring pad group 102, are arranged in a convex shape like the pad group 102, and from the group of the plurality of transducer wiring pads 302, a group of a plurality of signal patterns extend toward a proximal end side of the ultrasound transducer printed circuit board 301.

On one side of the ultrasound transducer printed circuit board 301, a first flexible printed circuit board wiring pad group 307 is arranged in a longitudinal axis direction of the ultrasound transducer printed circuit board 301.

Meanwhile, on the other side of the ultrasound transducer printed circuit board 301, a second flexible printed circuit board wiring pad group 308 is arranged in the longitudinal axis direction of the ultrasound transducer printed circuit board 301.

All of the plurality of signal pattern groups once extend in the longitudinal axis direction of the ultrasound transducer printed circuit board 301 toward a proximal end portion 303, then bend and extend toward the first flexible printed circuit board wiring pad group 307 or the second flexible printed circuit board wiring pad group 308 provided on either of the both sides, and are connected thereto.

To the first flexible printed circuit board wiring pad group 307 and the second flexible printed circuit board wiring pad group 308, a relay flexible printed circuit board for changing a direction that plays the same role as the second relay flexible printed circuit board 121 in the first embodiment is connected, though not shown, and as in the first and second embodiments, the relay flexible printed circuit board is properly connected to a coaxial signal cable provided in the longitudinal axis direction of the ultrasound transducer printed circuit board 301.

As described above, in the ultrasound endoscope of the third embodiment,
(1) the plurality of signal patterns extending from the plurality of ultrasound transducers are divided into the plurality of groups on the ultrasound transducer printed circuit board,
(2) all of the signal pattern groups once extend in the longitudinal axis direction of the ultrasound transducer printed circuit board and then bend at a predetermined angle, the pad electrode sections in the distal end portions are arranged in the longitudinal axis direction of the board, and the directions of the signal lines extending from the pad electrode sections substantially in the short axis direction are changed into the longitudinal axis direction by the direction changing board.

This allows a further reduction in diameter of the ultrasound probe and the ultrasound endoscope as compared with the first and second embodiment depending on the shape of the ultrasound transducer printed circuit board.

Also in the third embodiment, the board may be configured as a double-sided board as in the first embodiment.

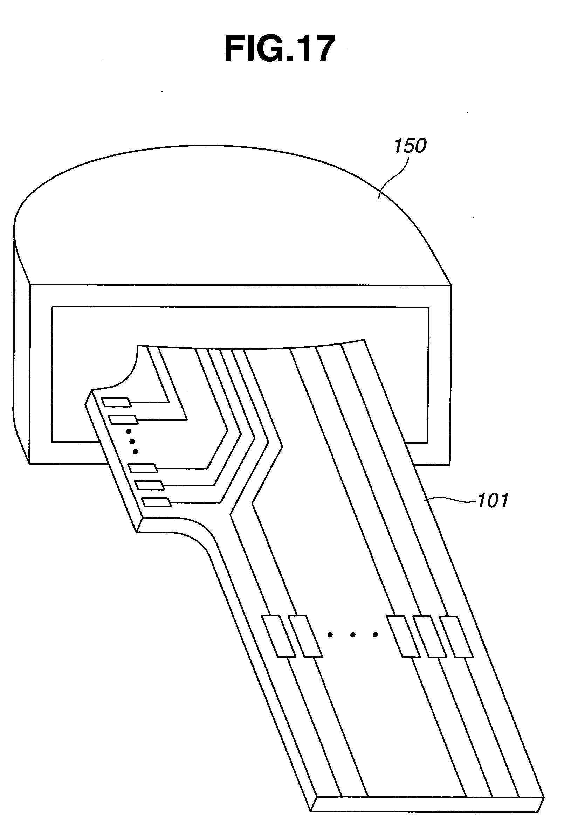

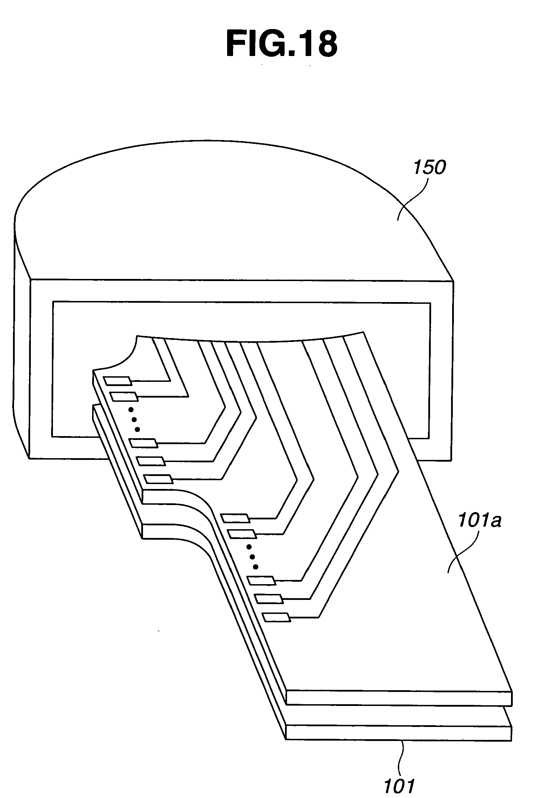

Further, in the above described embodiments, the example of using one ultrasound transducer printed circuit board is described, but not being limited thereto, a plurality of ultrasound transducers printed circuit boards may be used in combination. In this case, for example, when one ultrasound transducer printed circuit board 101 is used, the distal end portion of the ultrasound transducer printed circuit board 101 engages an ultrasound probe unit 150 as shown in Fig. 17, while when two ultrasound transducer printed circuit boards 101 and 101a are used, the ultrasound transducer printed circuit boards 101 and 101 a are provided to be stacked in a plane direction, and distal end portions thereof engage the ultrasound probe unit 150 as shown in Fig. 18.

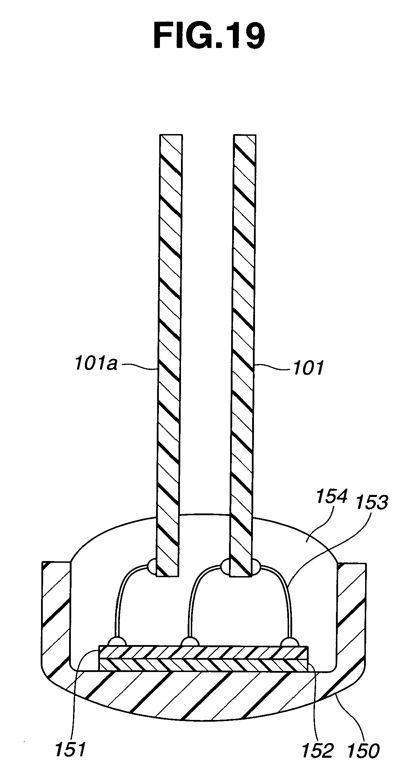

At this time, when the ultrasound transducer printed circuit board 101 is a double-sided board, while the ultrasound transducer printed circuit board 101a is a single-sided board among the two ultrasound transducer printed circuit boards 101 and 101a, a transducer wiring pad group of each thereof and an ultrasound transducer (piezoelectric element) 151 are connected by a wire 153 as shown in Fig. 19. In Fig. 19, reference numerals 152, 154 and 155 denote an acoustic matching layer, a packing material, and an acoustic lens.

In the present embodiments, the ultrasound probe including the transducers arranged in the convex shape that has a significant advantage in reduction in diameter, and the ultrasound endoscope including the ultrasound probe are described as an example, but the same advantage can be obtained by transducers arranged in line or two-dimensionally.

The present application claims priority of Japanese Patent Application No. 2006-162938 filed in Japan on June 12, 2006.

### Industrial Applicability

According to the present invention, an ultrasound probe and an ultrasound endoscope including an ultrasound probe can be provided that allow a reduction in diameter of the ultrasound probe and the ultrasound endoscope and allow reliable and easy electrical connection of signal lines extending from ultrasound transducers.

## Claims

1. An ultrasound probe (12) comprising:
a substantially rectangular-shaped ultrasound transducer printed circuit board (101) having a distal end portion formed in a projecting arcuate shape, and provided with a first electrode group (102), a second electrode group (108), and a first signal pattern group (105);
a plurality of ultrasound transducers (9) arranged in the arcuate shape in the distal end portion of the ultrasound transducer printed circuit board (101); and
a signal pattern direction changing printed circuit board (121) provided with a second signal pattern group (122) and a third electrode group (123), wherein
the signal pattern direction changing printed circuit board (121) is completely overlapping an area of the ultrasound transducer printed circuit board (101);
the first electrode group (102) is constituted by a plurality of first electrode sections (102) provided in the arcuate shape in the distal end portion of the ultrasound transducer printed circuit board (101), each of the first electrode sections (102) facing and being connected to each of the plurality of ultrasound transducers (9);
the second electrode group (108) is constituted by a plurality of second electrode sections (108) arranged in a longitudinal axis direction of the ultrasound transducer printed circuit board (101);
the first signal pattern group (106) is constituted by a plurality of first signal patterns (106) adapted to connect between the second electrode sections (108) and a part of the first electrode sections (102), wherein the first signal pattern group (106) extends from the part of the first electrode sections (102) towards a proximal end side of the ultrasound transducer printed circuit board (101), then bends in an angle direction different from the longitudinal axis direction, and further extends toward each electrode section of the second electrode group (108);
the third electrode group (123) is constituted by a plurality of third electrode sections (123) arranged on a proximal end portion side of the signal pattern direction changing printed circuit board (121) in a short axis direction of the signal pattern direction changing printed circuit board (121); and
the second signal pattern group (122) is constituted by a plurality of second signal patterns (122) adapted to connect between the second electrode sections (108) and the third electrode sections (123) and has one end connectable to each electrode section of the second electrode sections (108), extends from the one end in an angle direction different from the longitudinal axis direction, then bends in an angle direction different from the extending direction, and further extends in the longitudinal axis direction toward the proximal end portion.

2. The ultrasound probe (12) according to claim 1, wherein the signal pattern direction changing printed circuit board (121) is adapted to overlap within a short axis dimension of the ultrasound transducer printed circuit (101).

3. The ultrasound probe (12) according to claim 1, wherein
a cable (124) connected to the third electrode group (123) is arranged within a short axis dimension of the ultrasound transducer printed circuit board (101).

4. The ultrasound probe (12) according to claim 1, wherein
the ultrasound transducer printed circuit board (101) further includes a third signal pattern group (105) and a fourth electrode group (107),
the fourth electrode group (107) is constituted by a plurality of fourth electrode sections (107) arranged on a proximal end portion side of the ultrasound transducer printed circuit board (101) in a short axis direction of the ultrasound transducer printed circuit board (101), and
the third signal pattern group (105) is constituted by a plurality of third signal patterns (105) adapted to connect between the first electrode sections (102) other than the part of the first electrodes (102) connected to the first signal pattern (105) and the fourth electrode sections (107).

5. The ultrasound probe (12) according to claim 4, wherein a cable (114) connected to the fourth electrode group (107) is arranged within a short axis dimension of the ultrasound transducer printed circuit board (101).

6. The ultrasound probe (12) according to any one of claims 1 to 5, wherein two or more signal pattern direction changing printed circuit boards (121) are provided.

7. An ultrasound endoscope (1), comprising the ultrasound probe (12) according to any one of claims 1 to 6 at a distal end (2a) of an insertion section of the endoscope.

## Patentansprüche

1. Ultraschallsonde (12), die umfasst:
eine im Wesentlichen rechteckig geformte bedruckte Ultraschallwandler-Leiterplatte (101), die einen in einer vorspringenden Kreisbogenform ausgebildeten distalen Endabschnitt hat und mit einer ersten Elektrodengruppe (102), einer zweiten Elektrodengruppe (108) und einer ersten Signalmustergruppe (105) versehen ist;
eine Mehrzahl von Ultraschallwandlern (9), die in der Kreisbogenform in dem distalen Endabschnitt der bedruckten Ultraschallwandler-Leiterplatte (101) angeordnet sind; und
eine bedruckte Signalmusterrichtungsänderungs-Leiterplatte (121), die mit einer zweiten Signalmustergruppe (122) und einer dritten Elektrodengruppe (123) versehen ist, wobei
die bedruckte Signalmusterrichtungsänderungs-Leiterplatte (121) eine Fläche der bedruckten Ultraschallwandler-Leiterplatte (101) vollständig überlappt;
die erste Elektrodengruppe (102) durch eine Mehrzahl von ersten Elektrodenabschnitten (102) gebildet wird, die in der Kreisbogenform in dem distalen Endabschnitt der bedruckten Ultraschallwandler-Leiterplatte (101) vorgesehen sind, wobei jeder der ersten Elektrodenabschnitte (102) jedem der Mehrzahl von Ultraschallwandlern (9) zugewandt und damit verbunden ist;
die zweite Elektrodengruppe (108) durch eine Mehrzahl von zweiten Elektrodenabschnitten (108) gebildet wird, die in Richtung einer Längsachse der bedruckten Ultraschallwandler-Leiterplatte (101) angeordnet sind;
die erste Signalmustergruppe (106) durch eine Mehrzahl von ersten Signalmustern (106) gebildet wird, die dazu eingerichtet sind, eine Verbindung zwischen den zweiten Elektrodenabschnitten (108) und einem Teil der ersten Elektrodenabschnitte (102) herzustellen, wobei sich die erste Signalmustergruppe (106) von einem Teil der ersten Elektrodenabschnitte (102) in Richtung einer proximalen Endseite der bedruckten Ultraschallwandler-Leiterplatte (101) erstreckt, sich dann in eine Winkelrichtung biegt, die sich von der Richtung der Längsachse unterscheidet, und sich weiter in Richtung eines jeden Elektrodenabschnitts der zweiten Elektrodengruppe (108) erstreckt;
die dritte Elektrodengruppe (123) durch eine Mehrzahl von dritten Elektrodenabschnitten (123) gebildet wird, die auf einer proximalen Endabschnittsseite der bedruckten Signalmusterrichtungsänderungs-Leiterplatte (121) in Richtung einer kurzen Achse der bedruckten Signalmusterrichtungsänderungs-Leiterplatte (121) angeordnet sind; und
die zweite Signalmustergruppe (122) durch eine Mehrzahl von zweiten Signalmustern (122) gebildet wird, die dazu eingerichtet sind, eine Verbindung zwischen den zweiten Elektrodenabschnitten (108) und den dritten Elektrodenabschnitten (123) herzustellen, und ein Ende hat, das mit jedem Elektrodenabschnitt der zweiten Elektrodenabschnitte (108) verbindbar ist, sich von dem einen Ende in einer Winkelrichtung erstreckt, die sich von der Richtung der Längsachse unterscheidet, sich dann in eine Winkelrichtung biegt, die sich von der Erstreckungsrichtung unterscheidet, und sich weiter in Richtung der Längsachse des proximalen Endabschnitts erstreckt.

2. Ultraschallsonde (12) nach Anspruch 1, bei der die bedruckte Signalmusterrichtungsänderungs-Leiterplatte (121) dazu eingerichtet ist, innerhalb einer Dimension einer kurzen Achse der bedruckten Ultraschallwandler-Leiterplatte (101) zu überlappen.

3. Ultraschallsonde (12) nach Anspruch 1, bei der
ein Kabel (124), das mit der dritten Elektrodengruppe (123) verbunden ist, innerhalb einer Dimension einer kurzen Achse der bedruckten Ultraschallwandler-Leiterplatte (101) angeordnet ist.

4. Ultraschallsonde (12) nach Anspruch 1, bei der
die bedruckte Ultraschallwandler-Leiterplatte (101) ferner eine dritte Signalmustergruppe (105) und eine vierte Elektrodengruppe (107) umfasst,
die vierte Elektrodengruppe (107) durch eine Mehrzahl von vierten Elektrodenabschnitten (107) gebildet wird, die auf einer proximalen Endabschnittsseite der bedruckten Ultraschallwandler-Leiterplatte (101) in Richtung einer kurzen Achse der bedruckten Ultraschallwandler-Leiterplatte (101) angeordnet sind, und
die dritte Signalmustergruppe (105) durch eine Mehrzahl von dritten Signalmustern (105) gebildet wird, die dazu eingerichtet sind, eine Verbindung zwischen den ersten Elektrodenabschnitten (102) herzustellen, die sich von dem Teil der ersten Elektroden (102) unterscheiden, die mit den ersten Signalmustern (105) und den vierten Elektrodenabschnitten (107) verbunden sind.

5. Ultraschallsonde (12) nach Anspruch 4, bei der ein Kabel (114), das mit der vierten Elektrodengruppe (107) verbunden ist, innerhalb einer Dimension einer kurzen Achse der bedruckten Ultraschallwandler-Leiterplatte (101) angeordnet ist.

6. Ultraschallsonde (12) nach einem der Ansprüche 1 bis 5, bei der zwei oder mehr bedruckte Signalmusterrichtungsänderungs-Leiterplatten (121) vorgesehen sind.

7. Ultraschallendoskop (1), das die Ultraschallsonde (12) nach einem der Ansprüche 1 bis 6 an einem distalen Ende (2a) eines Einführabschnitts des Endoskops umfasst.

## Revendications

1. Sonde ultrasonore (12) comprenant :
une carte de circuit imprimé (101) de transducteurs ultrasonores de forme sensiblement rectangulaire comportant une partie d'extrémité distale formée selon une forme arquée saillante, et munie d'un premier groupe d'électrodes (102), d'un deuxième groupe d'électrodes (108), et d'un premier groupe de motifs de signaux (105) ;
une pluralité de transducteurs ultrasonores (9) agencés dans la forme arquée dans la partie d'extrémité distale de la carte de circuit imprimé (101) de transducteurs ultrasonores ; et
une carte de circuit imprimé (121) de changement de direction de motifs de signaux munie d'un deuxième groupe de motifs de signaux (122) et d'un troisième groupe d'électrodes (123), dans laquelle
la carte de circuit imprimé (121) de changement de direction de motifs de signaux chevauche complètement une zone de la carte de circuit imprimé (101) de transducteurs ultrasonores ;
le premier groupe d'électrodes (102) est constitué d'une pluralité de premières sections d'électrodes (102) prévues dans la forme arquée dans la partie d'extrémité distale de la carte de circuit imprimé (101) de transducteurs ultrasonores, chacune des premières sections d'électrodes étant en regard de et connectée à chacun de la pluralité de transducteurs ultrasonores (9) ;
le deuxième groupe d'électrodes (108) est constitué d'une pluralité de deuxièmes sections d'électrodes (108) agencées dans une direction d'axe longitudinal de la carte de circuit imprimé (101) de transducteurs ultrasonores ;
le premier groupe de motifs de signaux (106) est constitué d'une pluralité de premiers motifs de signaux (106) adaptés pour connexion entre les deuxièmes sections d'électrodes (108) et une partie des premières sections d'électrodes (102), dans laquelle le premier groupe de motifs de signaux (106) s'étend depuis la partie des premières sections d'électrodes (102) vers un côté d'extrémité proximale de la carte de circuit imprimé (101) de transducteurs ultrasonores, puis se courbe dans une direction angulaire différente de la direction d'axe longitudinal, et s'étend en outre vers chaque section d'électrodes du deuxième groupe d'électrodes (108) ;
le troisième groupe d'électrodes (123) est constitué d'une pluralité de troisièmes sections d'électrodes (123) agencées sur un côté de partie d'extrémité proximale de la carte de circuit imprimé (121) de changement de direction de motifs de signaux dans une direction d'axe court de la carte de circuit imprimé (121) de changement de direction de motifs de signaux ; et
le deuxième groupe de motifs de signaux (122) est constitué d'une pluralité de deuxièmes motifs de signaux (122) adaptés pour connexion entre les deuxièmes sections d'électrodes (108) et les troisièmes sections d'électrodes (123) et comporte une extrémité pouvant être connectée à chaque section d'électrodes parmi les deuxièmes sections d'électrodes (108), s'étend depuis une extrémité dans une direction angulaire différente de la direction d'axe longitudinal, puis se courbe dans une direction angulaire différente de la direction d'extension, et s'étend en outre dans la direction d'axe longitudinal vers la partie d'extrémité proximale.

2. Sonde ultrasonore (12) selon la revendication 1, dans laquelle la carte de circuit imprimé (121) de changement de direction de motifs de signaux est adaptée pour chevauchement à l'intérieur d'une dimension d'axe court de la carte de circuit imprimé (101) de transducteurs ultrasonores.

3. Sonde ultrasonore (12) selon la revendication 1, dans laquelle
un câble (124) connecté au troisième groupe d'électrodes (123) est agencé à l'intérieur d'une dimension d'axe court de la carte de circuit imprimé (101) de transducteurs ultrasonores.

4. Sonde ultrasonore (12) selon la revendication 1, dans laquelle
la carte de circuit imprimé (101) de transducteurs ultrasonores comprend en outre un troisième groupe de motifs de signaux (105) et un quatrième groupe d'électrodes (107),
le quatrième groupe d'électrodes (107) est constitué d'une pluralité de quatrièmes sections d'électrodes (107) agencées sur un côté de partie d'extrémité proximale de la carte de circuit imprimé (101) de transducteurs ultrasonores dans une direction d'axe court de la carte de circuit imprimé (101) de transducteurs ultra-sonores, et
le troisième groupe de motifs de signaux (105) est constitué d'une pluralité de troisièmes motifs de signaux (105) adaptés pour connexion entre les premières sections d'électrodes (102) autres que la partie des premières électrodes (102) connectées aux premiers motifs de signaux (105) et les quatrièmes sections d'électrodes (107).

5. Sonde ultrasonore (12) selon la revendication 4, dans laquelle un câble (114) connecté au quatrième groupe d'électrodes (107) est agencé à l'intérieur d'une dimension d'axe court de la carte de circuit imprimé (101) de transducteurs ultrasonores.

6. Sonde ultrasonore (12) selon l'une quelconque des revendications 1 à 5, dans laquelle deux ou plus cartes de circuit imprimé (121) de changement de direction de motifs de signaux sont prévues.

7. Endoscope ultrasonore (1), comprenant la sonde ultrasonore (12) selon l'une quelconque des revendications 1 à 6 au niveau d'une extrémité distale (2a) d'une section d'insertion de l'endoscope.
